# EUROPÄISCHE PATENTSCHRIFT

(11) **EP 1 275 408 B1**
(45) Veröffentlichungstag und Bekanntmachung des Hinweises auf die Patenterteilung: **20.10.2004**
(21) Anmeldenummer: 02022117.2
(22) Anmeldetag: 01.08.1997
(51) Int. Cl.: A61M 1/16, B01D 61/30, F04C 13/00, F04C 15/04

(54) **Dialysegerät zur Entfernung von toxischen Substanzen aus Blut mit Mitteln zur Entkalkung eines Dialysierflüssigkeitskreislaufs sowie Verfahren zur Bestimmung des Verkalkungsgrades eines Dialysegerätes**
Dialysis machine with means for decalcification of the dialysate circuit and method for determining its degree of calcification
Appareil de dialyse avec moyens de détartrage du circuit de dialysat et méthode pour déterminer son niveau de calcification

(30) Priorität: 06.09.1996 DE 19636255
(43) Veröffentlichungstag der Anmeldung: 15.01.2003
(62) Teilanmeldung aus: 97113346.7
(73) Patentinhaber: Fresenius Medical Care Deutschland GmbH, 61352 Bad Homburg v.d.H. (DE)
(72) Erfinder: Spickermann, Reiner, 97535 Wasserlosen/ Burghausen (DE)
(74) Vertreter: Laufhütte, Dieter, Dr.-Ing.

(56) Entgegenhaltungen:
- EP-A- 0 366 950
- EP-A- 0 648 508
- US-A- 4 153 554

## Beschreibung

Die vorliegende Erfindung betrifft ein Dialysegerät zur Entfernung von toxischen Substanzen aus Blut mit Mitteln zur Entkalkung eines Dialysierflüssigkeitskreislaufs. Die Erfindung betrifft ferner Verfahren zur Bestimmung des Verkalkungsgrades eines Dialysegeräts.

Bei der Bicarbonatdialyse zur Entfernung von toxischen Substanzen aus Blut tritt das Problem auf, dass Bicarbonat mit Calcium einen schwerlöslichen Niederschlag bildet. Die Komponenten werden daher bei der Bicarbonatdialyse als Natriumbicarbonat in der basischen Komponente, und als Calciumchlorid, Bestandteil der sauren Komponente, getrennt gehalten, und erst in der Maschine selbst oder kurz vor Anwendung gemischt.

Trotz dieser Maßnahmen bilden sich in der Dialysemaschine Kristallisationskeime, was mit der Zeit zu einer stärkeren Ablagerung von Calciumcarbonat führt. Aus diesem Grunde ist es notwendig, eine Dialysemaschine von Zeit zu Zeit zu entkalken. Übliche Entkalkungsmittel sind beispielsweise Essigsäure oder Zitronensäure.

Aus der US 5,326,476 ist es bekannt, einen manuell bedienbaren bzw. zeitgesteuerten Entkalkungszyklus einzuleiten. Aus der EP 0 648 508 ist eine Wartungseinrichtung für ein Dialysegerät bekannt, in die der Benutzer vor Durchführung eines Wartungsvorgangs, wie beispielsweise der Desinfektion oder Entkalkung, eine einen entsprechenden Wirkstoff enthaltende Tablette einführt. Die Art der Tablette wird von der Einrichtung erkannt und der entsprechende Desinfektions- oder Entkalkungsvorgang wird nach dem Auflösen der Tablette oder nach Beendigung eines vorausgegangenen Wartungsvorgangs eingeleitet.

Diese Entkalkungsverfahren erweisen sich insofern als nachteilig, als eine manuelle Auswahl eines Entkalkungsprogramms durch das Bedienpersonal vorgenommen werden muss, was sich in der Regel als sehr zeitaufwendig gestaltet. Ferner besteht bei der zeitgesteuerten Entkalkung der Nachteil, dass eine Entkalkung auch dann vorgenommen wird, wenn der Verkalkungsgrad eine derartige Entkalkung noch gar nicht erforderlich machen würde. Dies führt zu einer unnötigen Beanspruchung der Entkalkungsvorrichtung. Außerdem steht das Dialysegerät zur eigentlichen Behandlung während dieses Zeitraumes nicht zur Verfügung.

Aufgabe der Erfindung ist daher die Schaffung eines Dialysegeräts mit einer Entkalkungsvorrichtung, welche die obigen Nachteile vermeidet.

Die Erfindung hat ferner zum Ziel, Verfahren anzugeben, die in vorteilhafter Weise zur Bestimmung des Verkalkungsgrades eines Dialysegeräts verwendet werden können.

Erfindungsgemäß wird diese Aufgabe gelöst durch ein Dialysegerät mit den Merkmalen des Patentanspruchs 1 sowie durch ein Verfahren mit den Merkmalen des Patentanspruchs 4. Bevorzugte Ausgestaltungen sind Gegenstand der Unteransprüche.

Mit dem erfindungsgemäßen Dialysegerät wird eine Entkalkung lediglich in Abhängigkeit vom tatsächlich gemessenen Verkalkungszustand automatisch initiiert. Eine Beanspruchung des Bedienpersonals, sowie eine unnötige Betätigung der Entkalkungsmittel kann somit vermieden werden.

Gemäß der vorliegenden Erfindung ist der Verkalkungsgrad der Dialysemaschine mittels charakteristischer Parameter einer Pumpe, insbesondere einer Zahnradpumpe, welche zur Förderung von Dialysierflüssigkeit durch die Dialysiermaschine dient, bestimmbar. Dieses Bestimmungsverfahren lässt sich in sehr einfacher und zuverlässiger Weise realisieren, da bestimmte charakteristische Parameter einer Zahnradpumpe durch zunehmende Verkalkung in bestimmter Weise verändert werden.

Als besonders vorteilhaft erweist sich, den Verkalkungsgrad über eine Änderung des von der Pumpe geförderten Dialysierflüssigkeitsflusses zu bestimmen.

Es ist gleichfalls möglich, den Verkalkungsgrad über eine Änderung der Pumpenspannung und/oder des Pumpenstromes zu bestimmen.

Gemäß einer vorteilhaften Weiterbildung weist das Dialysegerät Mittel auf, die bei Feststellung eines vorbestimmten Verkalkungsgrades diese Information speichern und die automatische Entkalkung erst nach Beendigung einer Dialyse einleiten. Hierdurch ist gewährleistet, dass der Dialysebetrieb in keiner Weise behindert wird.

Gemäß einer weiteren Ausgestaltung der Erfindung ist vorgesehen, die für eine Bicarbonatdialyse verwendete saure Komponente als Entkalkungsmittel zu verwenden. Hiermit ist eine besonders einfache Möglichkeit zur Entkalkung eine Dialysegeräts zur Verfügung gestellt.

Die Erfindung stellt ferner ein Verfahren zur Verfügung, bei dem wenigstens ein charakteristischer Parameter einer Pumpe, insbesondere einer Zahnradpumpe, welche zur Förderung von Dialyseflüssigkeit durch das Dialysegerät dient, bestimmt wird, und der derart ermittelte Parameterwert mit einem Verkalkungsgrad in Beziehung gesetzt wird. Es wurde nämlich festgestellt, dass sich die Betriebs- bzw. Leistungsparameter von Zahnradpumpen durch Verkalkung in einer bestimmten Weise ändern.

Es erweist sich als besonders vorteilhaft, als charakteristische Parameter den von der Zahnradpumpe geförderten Dialysierflüssigkeitsfluß, oder den Pumpenstrom oder die Pumpenspannung der Zahnradpumpe zu verwenden. Diese Parameter sind in einfacher und zuverlässiger Weise messbar, und in eindeutiger Weise mit dem aktuellen Verkalkungsgrad des Dialysiergeräts in Beziehung zu setzen.

Bevorzugte Ausführungsformen der Erfindung werden nun näher erläutert.

Erfindungsgemäß wird der Verkalkungsgrad eines Dialysegeräts durch Auswertung bestimmter Parameter einer zur Förderung der Dialysierflüssigkeit verwendeten Zahnradpumpe bestimmt.

Es ist bei Dialysegeräten bekannt, derartige Zahnradpumpen zur Förderung von Dialysierflüssigkeit einzusetzen. Durch die zunehmende Verkalkung bei einer Bicarbonatdialyse verändern sich charakteristische Parameter dieser Zahnradpumpe. Tritt diese Verkalkung beispielsweise bei Verwendung von Dialysegeräten mit einem Konstant-Spannungsbetrieb der Zahnrad- bzw. Förderpumpe auf, so äußert sich diese Verkalkung beispielsweise durch eine Zunahme des Dialysierflüssigkeitsflusses.

Bei Überschreitung einer gewissen Zunahme, beispielsweise um 20%, ist es dann möglich, einen Entkalkungszyklus automatisch zu initiieren. Es ist auch hier gleichfalls möglich ein Alarmsignal zu erzeugen und beispielsweise ein Flag- oder ein Triggersignal für einen Entkalkungszyklus zu setzen bzw. zu erzeugen, welche die tatsächliche Entkalkung beispielsweise erst nach Beendigung der Dialyse einleitet. Es ist gleichfalls möglich, zur Bestimmung des Verkalkungsgrades andere Pumpenparameter zu verwenden, beispielsweise, je nach verwendeter Pumpensteuerung, die Zu- bzw. Abnahme des Stroms und/oder der Spannung der Pumpe.

Beiden Ausführungsformen ist gemeinsam, dass sie Mittel aufweisen, über welche bei Feststellung eines vorbestimmten Verkalkungsgrades des Dialysiergeräts eine automatische Entkalkung eingeleitet wird. Hierbei sind beispielsweise Entkalkungsmittel wie Essigsäure, Peressigsäure oder Zitronensäure verwendbar.

Gemäß einer bevorzugten Ausführungsform wird die bei der Bicarbonatdialyse verwendete saure Komponente auch zur Entkalkung verwendet. Dies ist möglich, da in dieser Komponente meist Acetat enthalten ist. Hiermit können komplette Desinfektionszyklen mit anderen kalklösenden Desinfektionsmitteln weitgehend vermieden werden.

Wesentliche Vorteile sind hier, dass weder ein zusätzliches Reservoir für das Entkalkungsmittel noch eine zusätzliche Vorrichtung zur Förderung des Entkalkungsmittels benötigt wird. Ferner ist vor Beginn des Entkalkungszyklus keine Konnektion bzw. Änderung einer Konnektion am Dialysegerät vorzunehmen, da die saure Komponente bereits am Dialysegerät angeschlossen ist, sei es über Versorgung mittels Kanister oder über eine zentrale Versorgungseinrichtung. Das Dialysegerät kann dann bei Vorliegen der o.g. Kriterien für eine Entkalkung beispielsweise nach Erkennen des Zustandes "Dialyseende" vollautomatisch einen Entkalkungszyklus einleiten.

Erfindungsgemäß ist daher eine Entkalkung eines Dialysegeräts derart vereinfacht, dass eine einfache, bedienerfreundlich und sichere Regenerierung der Dialysemaschine gewährleistet ist. Hierbei ist sowohl der Erfolg der Entkalkung selbst, als auch die Patientensicherheit gewährleistet.

Zur weiteren Erhöhung der Sicherheit ist es möglich, Mittel vorzusehen, die nach Beendigung eines Entkalkungszyklus in der Lage sind, Reste des verwendeten Entkalkungsmittels zu detektieren, so dass das Verbleiben von Entkalkungsmittelresten in dem Dialysegerät wirksam vermieden werden kann.

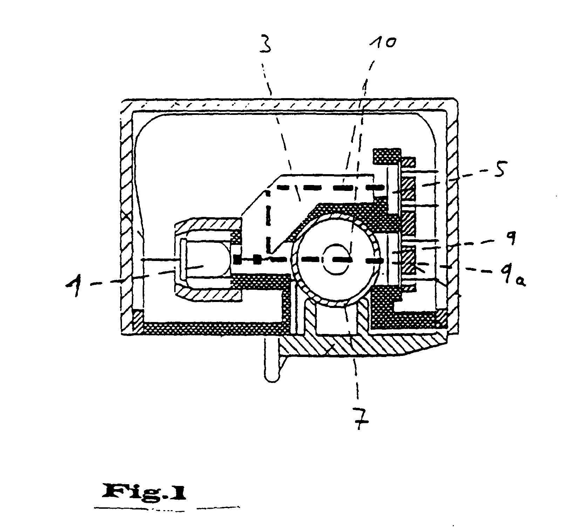

## Patentansprüche

1. Dialysegerät zur Entfernung von toxischen Substanzen aus biologischen Flüssigkeiten mit Mitteln zur Entkalkung eines Dialysierflüssigkeitskreislaufs,
und mit Mitteln, über welche bei Feststellung eines vorbestimmten Verkalkungsgrades des Dialysegeräts eine automatische Entkalkung des Dialysierflüssigkeitskreislaufs des Dialysegeräts einleitbar ist,
**dadurch gekennzeichnet,**
**dass** Mittel zur Bestimmung des Verkalkungsgrades vorgesehen sind, die den Verkalkungsgrad des Dialysegerätes über die Bestimmung charakteristischer Parameter einer Pumpe, insbesondere einer Zahnradpumpe, welche zur Förderung von Dialysierflüssigkeit durch das Dialysegerät dient, ermitteln.

2. Dialysegerät nach Anspruch 1, **dadurch gekennzeichnet, dass** der Verkalkungsgrad über eine Änderung des von der Pumpe geförderten Dialysierflüssigkeitsflusses bestimmbar ist.

3. Dialysegerät nach Anspruch 1, **dadurch gekennzeichnet, dass** der Verkatkungsgrad über eine Änderung des Pumpenstromes und/oder der Pumpenspannung bestimmbar ist.

4. Verfahren zur Bestimmung des Verkalkungsgrades eines Dialysegeräts nach einem der Ansprüche 1-3, **dadurch gekennzeichnet, dass** wenigstens ein charakteristischer Parameter einer Pumpe, insbesondere einer Zahnradpumpe, welche zur Förderung von Dialyseflüssigkeit durch das Dialysegerät dient, bestimmt wird und der derart ermittelte Parameterwert mit einem Verkalkungsgrad des Dialysegeräts in Beziehung gesetzt wird.

5. Verfahren nach Anspruch 4, **dadurch gekennzeichnet, dass** als charakteristischer Parameter ein von der Pumpe geförderter Dialysierflüssigkeitsfluß und/oder der Pumpenstrom und/oder die Pumpenspannung verwendet wird.

## Claims

1. A dialysis apparatus for the removal of toxic substances from biological fluids comprising means for the decalcification of a dialysing fluid circuit, and means by way of which automatic decalcification of the dialysing fluid circuit of the dialysis apparatus can be initiated when a predetermined degree of calcification of the dialysis apparatus is detected,
**characterised in that**
there are provided means for determining the degree of calcification, which ascertain the degree of calcification of the dialysis apparatus by way of determining characteristic parameters of a pump, in particular a gear pump, which serves to convey dialysing fluid through the dialysis apparatus.

2. A dialysis apparatus according to claim 1 **characterised in that** the degree of calcification can be determined by way of a change in the flow of dialysing fluid conveyed by the pump.

3. A dialysis apparatus according to claim 1 **characterised in that** the degree of calcification can be determined by way of a change in the pump current and/or the pump voltage.

4. A method of determining the degree of calcification of a dialysis apparatus according to one of claims 1 to 3 **characterised in that** at least one characteristic parameter of a pump, in particular a gear pump, which serves to convey dialysis fluid through the dialysis apparatus is determined and the parameter value ascertained **in that** way is related to a degree of calcification of the dialysis apparatus.

5. A method according to claim 4 **characterised in that** a flow of dialysing fluid conveyed by the pump and/or the pump current and/or the pump voltage is used as the characteristic parameter.

## Revendications

1. Appareil de dialyse servant à éliminer des substances toxiques à partir de fluides biologiques, muni de moyens de détartrage d'un circuit de liquide de dialyse et muni de moyens permettant de déclencher un détartrage automatique du circuit de liquide de dialyse lorsqu'un degré d'entartrage prédéterminé de l'appareil de dialyse est constaté, **caractérisé en ce que**, pour déterminer le degré d'entartrage, il est prévu des moyens qui détectent le degré d'entartrage de l'appareil de dialyse par le biais de la détermination de paramètres caractéristiques d'une pompe, en particulier d'une pompe à engrenage, servant à alimenter du liquide de dialyse dans l'appareil de dialyse.

2. Appareil de dialyse selon la revendication 1, **caractérisé en ce que** le degré d'entartrage peut être déterminé à partir d'une variation du débit de liquide de dialyse alimenté par la pompe.

3. Appareil de dialyse selon la revendication 1, **caractérisé en ce que** le degré d'entartrage peut être déterminé à partir d'une variation de l'intensité de la pompe et/ou de la tension de la pompe.

4. Procédé de détermination du degré d'entartrage d'un appareil de dialyse selon l'une des revendications 1 à 3, **caractérisé en ce que** l'on détermine au moins un paramètre caractéristique d'une pompe, en particulier d'une pompe à engrenage, servant à alimenter du liquide de dialyse dans l'appareil de dialyse et **en ce que** la valeur ainsi déterminée du paramètre est corrélée avec un degré d'entartrage de l'appareil de dialyse.

5. Procédé selon la revendication 4, **caractérisé en ce que** l'on utilise comme paramètre caractéristique un débit de liquide de dialyse alimenté par la pompe et/ou l'intensité de la pompe et/ou la tension de la pompe.
